(19) Europäisches Patentamt
European Patent Office
Office européen des brevets

(11) Publication number: **0 010 296**
**B1**

(12) # EUROPEAN PATENT SPECIFICATION

(45) Date of publication of patent specification: 01.12.82

(51) Int. Cl.³: C 12 Q 1/00, G 01 N 33/52

(21) Application number: 79104011.6

(22) Date of filing: 17.10.79

(54) **Test composition for the determination of triglycerides and its use.**

(30) Priority: 18.10.78 JP 127328/78

(43) Date of publication of application:
30.04.80 Bulletin 80/9

(45) Publication of the grant of the patent:
01.12.82 Bulletin 82/48

(84) Designated Contracting States:
DE FR GB IT

(56) References cited:
DE - B - 2 459 087
FR - A - 2 387 992

RESEARCH DISCLOSURE, no. 161, September 1977, pages 94—98 Vant. Hants, G.B. "Integral Element for the detection of glycerol triglycerides"

(73) Proprietor: KYOWA HAKKO KOGYO CO., LTD
Ohtemachi Bldg. Ohtemachi 1-chome Chiyoda-ku
Tokyo (JP)

(72) Inventor: Nagai, Toshiaki
935-32, Shimonagakubo Nagaizumi-cho
Sunto-gun Shizuoka-ken (JP)
Inventor: Terada, Osamu
1-15-8, Tamagawagakuen Machida-shi
Tokyo (JP)
Inventor: Uwajima, Takayuki
4-5-10, Naka-machi Machida-shi
Tokyo (JP)
Inventor: Mihara, Akira
Kyowa Apartment House 3-9-10 Naka-Machi
Machida-shi Tokyo (JP)
Inventor: Aisaka, Kazuo
941, Honmachida Machida-shi
Tokyo (JP)
Inventor: Akita, Hiroko
229, Nakakibogaoka Asahi-ku
Yokohama-shi Kanagawa-ken (JP)
Inventor: Shimizu, Yoshiaki
1188, Shimotogari Nagaizumi-cho
Sunto-gun Shizuoka-ken (JP)

(74) Representative: Vossius-Vossius-Tauchner-Heunemann-Rauh Patentanwälte
P.O.Box 860767 Siebertstrasse 4
D-8000 München 86 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

# Test composition for the determination of triglycerides and its use.

## Background of the Invention

The present invention relates to a method for the quantitative determination of triglycerides in a sample by an enzymatic reaction and a test composition suitable therefor.

The determination of triglycerides is a clinically significant lipid test for hyper-lipoidemia. However, the conventional methods for such determination are burdensome in operation and are, therefore, inconvenient for routine use.

For example, the most commonly employed procedure at present is the acetylacetone test in which triglycerides are first isolated by extraction with an organic solvent. The triglycerides are then hydrolyzed with an alkali and the glycerol which is formed is oxidized with an oxidizing agent such as sodium metaperiodate. The formaldehyde thus formed is condensed with acetylacetone and the degree of the color developed is determined by colorimetry. The operation thus comprises several steps. The acetylacetone procedure is disclosed in, for example, M.J. Fletcher, "A colorimetric method for estimating serum triglycerides" Clinica Chimica Acta Vol. 22, 393—397 (1968).

More recently, a method for the determination of serum triglycerides by an enzymatic reaction is disclosed in U.S.—A— 4,038,146. In this method, triglycerides are hydrolyzed into fatty acids and glycerol by the action of lipoprotein lipase and the glycerol is converted to dihydroxyacetone by the action of glycerol dehydrogenase in the presence of nicotinamide adenine dinucleotide (NAD). The NADH thus formed is converted to a pigment and the degree of color development in the invisible ray region is measured. Although this method is capable of being carried out in one step, it is difficult to adapt to automatic analytical instruments.

It has now been found that triglycerides in a sample may be quantitatively determined simply by the hydrolysis of triglycerides, the enzymatic oxidation of glycerol thus produced and thereafter detecting the products so formed or alternatively the consumption of oxygen.

## Summary of the Invention

According to the present invention, triglycerides in a sample are quantitatively determined by solvolysis of the triglycerides to form fatty acids and glycerol, oxidizing the glycerol with glycerol oxidase in the presence of oxygen to form glyceraldehyde and hydrogen peroxide and determining the amount of glyceraldehyde or hydrogen peroxide formed or determining the oxygen uptake. Broadly, the mechanism of the present invention is illustrated as follows:

Triglycerides

↓ Solvolysis

Fatty acid + Glycerol

↓ Enzymatic reaction (glycerol oxidase) in the presence of $O_2$

Glyceraldehyde + $H_2O_2$

↓ ↓

Detection Detection

The reactions described above proceed stoichiometrically and the amount of glyceraldehyde or hydrogen peroxide formed or oxygen uptake is proportional to the amount of triglyceride in the test sample.

## Detailed Description of the Invention

Basically, the method and test composition for the determination of the present invention comprises a system for converting triglycerides in a sample to glycerol and fatty acids (System I), a system for oxidizing the glycerol with glycerol oxidase in the presence of oxygen to form glyceraldehyde and hydrogen peroxide (System II) and a system for determining the glyceraldehyde or hydrogen peroxide formed or oxygen uptake (System III).

System I is carried out by applying conventional methods such as by solvolysis using an enzyme or an alkali. Typical enzymes include lipoprotein lipase (hereinafter referred to as LPL), cholesterol esterase, lipase and protease.

Glycerol oxidase (hereinafter referred to as GOD) used in the enzymatic conversion (System II), is obtained from cell bodies of a microorganism belonging to the genus *Aspergillus, Neurospora* or *Penicillium* by extraction and purification as is disclosed in DE—A—2 817 087 filed April 19, 1978.

For ease of reference, typical methods thereof are hereinafter described in examples 1 and 2. Other GOD may be used if it catalizes the above reaction.

The determination of the hydrogen peroxide formed may be carried out according to conventional methods. Methods using peroxidase (hereinafter referred to as POD) or catalase are particularly suitable because the determination is carried out in one step.

In the method using peroxidase, hydrogen peroxide is reacted with a coloring reagent to form a pigment and the absorption of the reaction solution is measured at the visible ray region, i.e. 400—600 nm. Suitable coloring reagents for the determination of hydrogen peroxide using peroxidase are selected from 4-aminoantipyrine (hereinafter referred to as 4—AA) and phenol, 4—AA and dimethylaniline, 3-methyl-2-benzothiazolinone hydrazone (hereinafter referred to as MBTH) and O-toluidine, MBTH and N,N-dimethylaniline, O-dianisidine and 4—AA and N-ethyl-N-(3-methylphenyl)-N′-acetylethylenediamine.

In the method using catalase, hydrogen peroxide is reacted with methanol in the presence of catalase to form formaldehyde which is then reacted with acetylacetone in the presence of ammonia to form a yellow pigment, i.e., 3,5-diacetyl-1,4-dihydrolutidine, and the absorption of the reaction solution is measured colorimetrically.

In the determination of the glyceraldehyde formed, the glyceraldehyde is reacted with 2,4-dinitrophenylhydrazine in the presence of a strong alkali to form a colored substance, i.e. 2,4-dinitrophenylhydrazone and the absorption of the reaction solution is measured at 495 nm. In this method, the pH of the enzymatic reaction and the pH of the coloring reaction system are different and therefore, the determination cannot be carried out in one step.

In the determination of oxygen uptake, a sample containing triglycerides, a reagent for saponification of triglyceride and glycerol oxidase are dissolved in a buffer solution and the partial pressure of oxygen is measured by suitable known means.

In the determination of triglycerides in serum, when the serum contains triglycerides in high concentration and is turbid, the turbidity may be decreased by the addition of a surfactant in an amount of 0.1% such as Triton® x 100 (iso-octylphenoxy-poly-ethoxy ethanol containing ethylene oxide, product sold by Wako Junyaku Co., Ltd. and Rohm & Haas Co.) or Brij® 35 (polyoxyethylenelaurylether, Atlas Chemical Industries, Inc. and Wako Junyaku Co., Ltd.).

According to the present invention, the determination of triglycerides is performed by converting the triglycerides to acid and glycerol (solvolysis), enzymatically oxidizing the glycerol with glycerol oxidase (GOD) in the presence of oxygen to form glyceraldehyde and hydrogen peroxide and determining the amount of glyceraldehyde or hydrogen peroxide formed (e.g. by using POD or catalase) or the amount of oxygen consumed.

The enzymatic reactions in the present invention are carried out at 20—45°C, preferably 30—40°C and at pH 5—10 for 4—30 minutes.

The enzyme reagent of the present invention is preferably prepared by dissolving the enzyme(s) necessary for the determination in a buffer solution and freeze-drying the solution. The buffer solution is not limited except that the solution should exhibit the buffer action in the range of pH 6.0—9.5, and a preferred buffer solution is a 0.1M N-Tris (hydroxymethyl) methyl-2-aminoethane sulfonic acid (hereinafter referred to as TES) buffer solution (pH 8.0).

In the following Examples 1 and 2, production of GOD is illustrated.

### Example 1

In this example, *Aspergillus japonicus* KY—45 (ATCC 1042, NRRL 11102, FERM—P No. 3959) is inoculated in 300 ml of seed medium containing 10 g/l of glycerol, 10 g/l of malt extract, and 5 g/l of yeast extract (pH 6.2 before sterilization) in a 2 l Erlenmeyer flask, and cultured at 30°C with shaking for 48 hours. Nine hundred milliliters (corresponding to three flasks) of the resulting seed culture is inoculated in 15 l of the same medium as the seed medium in a 30 l jar fermenter, and cultured at 30°C with aeration (15 l/min.) and stirring (250 rpm) for 40 hours. The resulting culture broth is then filtered through a Buchner funnel, and the cakes are washed with water, whereby about 150 g (dry basis) of microbial cells is obtained.

The microbial cells are suspended in 5 l of 10 mM ammonium buffer solution (pH 8.0), and disrupted in a Dyno®-mill (made by Willy A. Bachofen, Switzerland). After disruption, the suspension is centrifuged with a freezing centrifuge (20,000 x G for 20 minutes), and 4.7 l of the supernatant (protein content: 51 g, specific activity: 0.05 units/mg) is obtained. The resulting supernatant is then admixed with ammonium sulfate, and fractions precipitated at 30—70% saturation with ammonium sulfate are collected and dissolved in 50 ml of a 10 mM ammonium buffer solution (pH 8.0). The resulting solution is dialyzed against 10 l of the 10 mM ammonium buffer solution, using a cellophane tube as a dialysis membrane.

Dialysis is continued for 48 hours with a total of 40 l of the dialysis solution, while exchanging the dialysis solution every 12 hours. Dialyzed enzyme solution is passed through a column (5.5 x 40 cm) of DEAE cellulose (made by Serva Co., West Germany) equilibrated in advance with 10 mM ammonium buffer solution (pH 8.0). The enzyme is adsorbed by this operation, and unadsorbed impure protein is washed out with the same buffer. Then, elution is carried out with a linear

gradient of 0.1M to 0.2M ammonium sulfate in 2 l of 10 mM ammonium buffer solution (pH 8.0), whereby glycerol oxidase is eluted. Five hundred milliliters of the active fractions (protein content: 1.2 g, specific activity 1.1) are collected, and admixed with ammonium sulfate to 70% saturation to precipitate the enzyme.

The precipitates are collected by centrifugation (20,000 x G for 20 minutes), and dissolved in 50 ml of a 10 mM ammonium buffer solution (pH 8.0). The solution is then passed through a column (5.5 x 80 cm) of Sephadex® G—100 (3-dimensional cross-linked dextran powder made by Pharmacia Fine Chemicals, Co., Sweden) equilibrated in advance with 10 mM ammonium buffer solution (pH 8.0). One liter of 10 mM ammonium buffer solution is passed through the column and the eluate is obtained in fractions. From the fractions, 300 ml of a fraction having a high specific activity (protein content: 310 mg, specific activity: 3.2) is obtained and admixed with ammonium sulfate to 70% saturation to precipitate the enzyme. Then, the precipitates are collected by centrifugation (20,000 x G for 20 minutes) and dissolved in 20 ml of 10 mM ammonium buffer solution (pH 8.0). The resulting solution is dialyzed for 24 hours against 5 l of 10 mM ammonium buffer solution (pH 8.0), using a cellophane tube as a dialysis membrane, while twice exchanging the dialysis solution. After the dialysis, the resulting enzyme solution is passed through a column (5.5 x 20 cm) of hydroxyapatite equilibrated in advance with 10 mM ammonium buffer solution (pH 8.0). An additional 250 ml of the same buffer is passed through the column, and the eluate is obtained in fractions. Fractions having a specific activity higher than 20 are collected and freeze-dried, whereby 10.2 mg powder of purified enzyme preparate of glycerol oxidase (specific activity: 30.2) is obtained. The purified enzyme has a specific activity about 610 times as high as that of the cell extract. The yield is 19% in terms of activity.

## Example 2

In this example, the same procedures as described in Example 1 are repeated except that *Neurospora crassa* KY—462 (NRRL 11106, FERM—P, No. 3960) is used, whereby 1.1 mg of a purified enzyme preparate of glycerol oxidase (specific activity: 14.1) is obtained in a 10.5% yield in terms of activity.

Practice of certain specific embodiments of the invention are illustrated by the following representative examples wherein the enzymes used are as follows:

Lipase and $\alpha$-chymotrypsin (protease) : products of Sei Kagaku Kogyo Co., Ltd. Japan.
GOD : The product obtained in Examples 1 or 2.
POD : Product of Washington Co., U.S.A.
LPL : Product obtained from the culture liquor of *Rhizopus japonicus* FERM p-3651.

## Example 3

In this example, Reagent 1 is prepared by dissolving the following components in 0.1M TES-buffer solution (pH 8.0) to make up 25 ml of an enzyme solution.

| | |
|---|---|
| Lipase | $6 \times 10^5$ IU |
| $\alpha$-Chymotrypsin | $2 \times 10^6$ IU |
| 4-aminoantipirine | 60 mg |

Then, 2.5 ml of the enzyme solution is poured into 10 ml-vials and is freeze-dried.

Reagent 2 is prepared by dissolving 500 IU of GOD and 1500 IU of POD in 0.1M TES-buffer solution (pH 8.0) to make up 25 ml of an enzyme solution. Then 2.5 ml of the enzyme solution is poured into 10 ml-vials and is freeze-dried.

A coloring solution is prepared by dissolving 300 mg of phenol and 300 mg of Triton x 100 in 0.1M TES-buffer solution (pH 8.0) to make up 25 ml of coloring solution.

A standard solution is also prepared by dissolving 26 mg of glycerol in 0.1M TES buffer solution (pH 8.0) to make up 100 ml of standard solution. The solution thus obtained contains 250 mg/dl of triolein. Unless otherwise indicated, the same standard is used in the following examples.

To carry out determination of triglyceride in a serum sample, the contents of a vial of Reagent-1 and a vial of Reagent-2 are dissolved in 30 ml of coloring solution to make up a test solution. Then 0.02 ml of serum is added to 3 ml of test solution and the mixture is incubated at 37°C for ten minutes. The absorption of the reaction solution at 505 nm is then measured. A calibration curve is prepared using the standard solution. Five samples of serum are tested according to the foregoing method and the results are shown below (table I).

TABLE I

| Serum No. | Degree of absorption (at 505 nm) | Triglyceride amount (mg/dl) |
|---|---|---|
| 1 | 0.053 | 148 |
| 3 | 0.075 | 196 |
| 5 | 0.088 | 247 |
| 7 | 0.095 | 267 |
| 9 | 0.105 | 295 |
| Standard | 0.089 | 250.0 |

Example 4

In this example, the components set forth below are dissolved in 25 ml of 0.1M TES-buffer solution (pH 6.8) to make up an enzyme reagent.

| | |
|---|---|
| LPL | 20,000 IU |
| GOD | 500 IU |
| POD | 1,000 IU |
| 4—AA | 60 mg |

After filtering, the solution is poured into 10 vials and freeze-dried.

To prepare a coloring solution, 350 mg of dimethylaniline is dissolved in 5 ml of water.

To prepare a buffer solution, 0.3 ml of Triton®X 100 is dissolved in 0.1M TES-buffer solution (pH 6.8) to make up 300 ml of solution.

To carry out determination of triglyceride in a serum sample, a vial of the enzyme reagent is dissolved in 30 ml of the buffer solution and 0.5 ml of coloring solution is added to the resultant solution to make up a test solution. Then, 0.02 ml of serum is added to 3 ml of test solution and incubated at 37°C for 10 minutes. Then, the absorption of the reaction solution at 550 nm is measured.

Five samples of serum is tested for the determination of triglyceride according to the foregoing method. The results are shown below (table II).

TABLE II

| Sample No. | Degree of Absorption | Amount of triglyceride (mg/dl) |
|---|---|---|
| 1 | 0.055 | 148 |
| 3 | 0.080 | 215 |
| 5 | 0.085 | 228 |
| 7 | 0.100 | 269 |
| 9 | 0.111 | 298 |
| Standard | 0.093 | 250.0 |

Example 5

In this example, an enzyme reagent is prepared by dissolving 50 IU of GOD and 50 mg of lactose in 10 ml of water and the solution is poured into 10 vials and freeze-dried.

A solution for hydrolysis is prepared by dissolving 5.6 g of potassium hydroxide in 100 ml of absolute ethanol.

A 0.1M ammonium buffer solution is prepared by mixing 96 ml of 0.1M ammonium chloride and 3 ml of 0.1N aqueous ammonia.

For the determination of triglyceride in a serum sample, 0.02 ml of serum is poured into a test tube and 0.1 ml of solution for hydrolysis is added thereto. The mixture is subjected to hydrolysis at 75°C for 10 minutes. Then, 0.1 ml of 1N hydrochloride and 3 ml of 0.1M ammonium buffer solution are added thereto and the mixture is heated at 37°C for 10 minutes. an enzymatic electrode (Beckmann 160C, Beckmann Co.) is equipped to the test tube. A vial of enzyme reagent is dissolved in 1 ml buffer solution and the enzyme solution is added to the test tube through a 0.1 ml microsyringe.

The change of oxygen uptake is then measured. A calibration curve is separately prepared using the standard solution.

Five samples of serum are tested in the foregoing manner and the results are shown below (table III).

TABLE III

| Sample No. | Ratio of partial pressure of oxygen* | Amount of triglyceride (mg/dl) |
|---|---|---|
| 1 | 0.861 | 147 |
| 3 | 0.816 | 194 |
| 5 | 0.769 | 245 |
| 7 | 0.740 | 275 |
| 9 | 0.724 | 292 |
| Standard | 0.764 | 250.0 |

* Ratio before the reaction is 1.000.

Example 6

In this example, an enzyme reagent is prepared by mixing the following components and pouring the mixture into five vials.

| | |
|---|---|
| Lipase | $6 \times 10^5$ IU |
| $\alpha$-chymotrypsin | $2.6 \times 10^{-6}$ IU |
| 4—AA | 60 mg |
| GOD | 500 IU |
| POD | 1,000 IU |
| D-Mannitol | 200 mg |

Then 300 mg of phenol and 300 mg of Triton® X 100 are dissolved in 0.1M ammonium buffer solution (pH 8.0) to make up 100 ml of coloring reagent.

To carry out determination of triglyceride in a serum sample, a vial of enzyme reagent is dissolved in a mixture of 20 ml of coloring reagent and 40 ml of water to make up a test solution. Then 20 $\mu$l samples of serum is added to 3 ml of the test solution and the reaction is carried out at 37°C for 5 minutes, after which the absorption at 505 nm is measured. The results for five samples are shown below (table IV).

TABLE IV

| Sample No. | Degree of absorption | Amount of triglyceride (mg/dl) |
|---|---|---|
| 1 | 0.021 | 146 |
| 3 | 0.029 | 201 |
| 5 | 0.036 | 250 |
| 7 | 0.040 | 278 |
| 9 | 0.043 | 299 |
| Standard | 0.036 | 250.0 |

Example 7

In this example, the following components are dissolved in 20 ml of water to make up an enzyme reagent.

| | |
|---|---|
| LPL | 20,000 IU |
| GOD | 500 IU |
| POD | 1,500 IU |
| 4—AA | 60 mg |
| Lactose | 40 mg |

Then 5 ml of the solution is pipetted into vials and freeze-dried.

To carry out determination of triglyceride in a sample, a vial of the enzyme reagent is dissolved in 75 ml of the same coloring reagent solution as in Example 6 to make up a test solution. Then 0.02 ml of serum is added to 3 ml of test solution and is incubated at 37°C for 10 minutes after which absorption at 505 nm is measured.

16 samples of serum are tested and the results are shown below. For comparison, the same serum samples are tested according to the known acetylacetone method, and the results are also set forth below (table V).

TABLE V

| Sample No. | Amount of triglyceride(mg/dl) | |
|---|---|---|
| | Acetylacetone method | Present method |
| 1 | 149 | 140 |
| 2 | 156 | 157 |
| 3 | 181 | 199 |
| 4 | 230 | 224 |
| 5 | 245 | 252 |
| 6 | 263 | 274 |
| 7 | 266 | 280 |
| 8 | 283 | 290 |
| 9 | 290 | 284 |
| 10 | 320 | 323 |
| 11 | 350 | 354 |
| 12 | 415 | 430 |
| 13 | 520 | 503 |
| 14 | 529 | 540 |
| 15 | 630 | 610 |
| 16 | 710 | 699 |

Example 8

In this example, the same procedures as described in Example 7 are repeated except that 60,000 IU of cholesterol esterase (product obtained from the culture liquor of *Pseudomonas fluorescens* ATCC 31156) having lipoprotein lipase activity is used instead of LPL. Five samples of serum (sample Nos. 1, 3, 5, 7 and 9) and standard solution are tested according to the foregoing method. The amounts (mg/dl) of triglyceride are 145, 197, 250, 278, 288 and 250, respectively.

**Claims**

1. A test composition for the determination of triglycerides in a sample, which comprises: a system for converting triglycerides in said sample to acids and glycerol; a system for oxidizing the glycerol with glycerol oxidase in the presence of oxygen to form glyceraldehyde and hydrogen peroxide; and a system for determining the amount of glyceraldehyde or hydrogen peroxide formed or the amount of oxygen consumed.

2. The test composition according to claim 1, wherein said system for converting triglycerides to fatty acids and glycerol comprises an enzyme selected from the group consisting of lipoprotein lipase, cholesterol esterase, lipase and protease.

3. The test composition according to claim 1, wherein said system for determination of the hydrogen peroxide formed comprises peroxidase and a coloring reagent.

4. A test composition for the determination of triglycerides in a sample which comprises an enzyme system selected from the group consisting of lipoprotein lipase, cholesterol esterase, lipase and protease; an oxidizing system consisting of glycerol oxidase; a determination system consisting of peroxidase, and a coloring reagent selected from the group of phenol and 4-aminoantipyrine, dimethylaniline and 4-aminoantipyrine, 3-methyl-2-benzothiazoline hydrazone and o-toluidine, 3-methyl-2-benzothiazolinone and N,N-dimethylaniline, o-dianisidine and 4-aminoantipyrine and N-ethyl-N-(3-methylphenyl)-N'-acetylethylenediamine.

5. Use of the test composition according to claims 1 to 4 for the quantitative determination of triglycerides.

**Revendications**

1. Composition réactive pour le dosage des triglycérides dans un échantillon, qui comprend: un système pour transformer les triglycérides dudit échantillon en acides et en glycérol; un système pour oxyder le glycérol avec l'oxydase du glycérol en présence d'oxygène pour fomer de l'aldéhyde glycérique et du peroxyde d'hydrogène; et un système pour doser la quantité d'aldéhyde glycérique ou de peroxyde d'hydrogène formée ou la quantité d'oxygène consommée.

2. Composition réactive selon la revendication 1, dans laquelle ledit système pour transformer les triglycérides en acides gras et en glycérol comprend un enzyme choisi dans le groupe formé par la lipase lipoprotéïque, l'estérase du cholestérol, la lipase et la protéase.

3. Composition réactive selon la revendication 1, dans laquelle ledit système pour le dosage du peroxyde d'hydrogène formé comprend la peroxydase et un réactif colorant.

4. Composition réactive pour le dosage des triglycérides dans un échantillon qui comprend un système enzymatique choisi dans le groupe formé par la lipase lipoprotéïque, l'estérase du cholestérol, la lipase et la protéase; un système oxydant consistant en l'oxydase du glycérol; un système de dosage consistant en la peroxydase, et un réactif colorant choisi dans le groupe phénol et 4-aminoantipyrine, diméthylaniline et 4-amino-antipyrine, 3-méthyl-2-benzothiazolinone-hydrazone et o-toluidine, 3-méthyl-2-benzothiazolinone et N,N-diméthylaniline, o-dianisidine et 4-amino-antipyrine et N-éthyl-N-(3-méthylphényl)-N'-acétyléthylénediamine.

5. Utilisation de la composition réactive selon les revendications 1 à 4 pour le dosage quantitatif des triglycérides.

**Patentansprüche**

1. Testzusammensetzung zur Bestimmung von Triglyceriden in einer Probe, dadurch gekennzeichnet, daß sie die folgenden Bestandsteile enthält: ein System zur Umwandlung von Triglyceriden in der Probe in Säure und Glycerin; ein System zur Oxidation des Glycerins mit Glycerinoxidase in Gegenwart von Sauerstoff unter Bildung von Glycerinaldehyd und Wasserstoffperoxid; sowie ein System zur Bestimmung der gebildeten Menge an Glycerinaldehyd oder Wasserstoffperoxid oder der Menge des verbrauchten Sauerstoffs.

2. Testzusammensetzung nach Anspruch 1, dadurch gekennzeichnet, daß das System zur Umwandlung der Triglyceride zu Fettsäuren und Glycerin ein Enzym aus der Grupe Lipoproteinlipase, Cholesterinesterase, Lipase und Protease enthält.

3. Testzusammensetzung nach Anspruch 1, dadurch gekennzeichnet, daß das System zur Bestimmung des gebildeten Wasserstoffperoxids Peroxidase und ein Färbemittel enthält.

4. Testzusammensetzung zur Bestimmung von Triglyceriden in einer Probe, dadurch gekennzeichnet, daß es die folgenden Bestandsteile enthält: ein Enzymsystem aus der Gruppe Lipoproteinlipase, Cholesterinesterase, Lipase und Protease; ein oxidierendes System aus Glycerinoxidase ; ein Nachweissystem aus Peroxidase und einem Färbemittel aus der Gruppe Phenol und 4-Aminoantipyrin, Dimethylanilin und 4-Aminoantipyrin, 3-Methyl-2-benzothiazolinon-hydrazon und o-Toluidin, 3-Methyl-2-benzothiazolinon und N,N-Dimethylanilin, o-Dianisidin und 4-Aminoantipyrin und N-Äthyl-N-(3-methylphenyl)-N'-acetyläthylendiamin.

5. Verwendung der Testzusammensetzung nach den Ansprüchen 1 bis 4 zur quantitativen Bestimmung von Triglyceriden.